(19) Europäisches Patentamt
European Patent Office
Office européen des brevets

(11) Publication number: **0 052 903**
**B1**

(12) **EUROPEAN PATENT SPECIFICATION**

(45) Date of publication of patent specification: **16.05.84**

(51) Int. Cl.$^3$: **C 07 C 61/40,** C 07 C 51/377

(21) Application number: **81201248.2**

(22) Date of filing: **04.11.81**

(54) **Preparation of dihalovinylcyclopropane carboxylic acids.**

(30) Priority: **26.11.80 GB 8037891**

(43) Date of publication of application:
**02.06.82 Bulletin 82/22**

(45) Publication of the grant of the patent:
**16.05.84 Bulletin 84/20**

(84) Designated Contracting States:
**AT BE CH DE FR GB IT LI NL SE**

(56) References cited:
**EP-A-0 021 521**
**GB-A-1 541 466**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Kramer, Petrus Anthonius**
**Badhuisweg 3**
**NL-1031 CM Amsterdam (NL)**

(74) Representative: **Hunter, Keith Roger Ian et al**
**4 York Road**
**London SE1 7NA (GB)**

EP 0 052 903 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the preparation of dihalovinylcyclopropane carboxylic acids.

US Patent Specification No. 4166063, discloses that reaction of a *cis*-hydroxy acid of the general formula

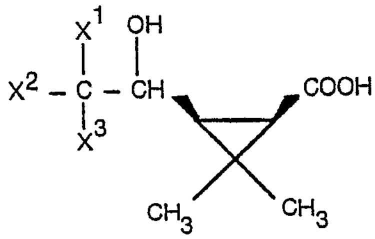

in which $X^1$, $X^2$ and $X^3$ when identical are selected from the group consisting of chlorine and bromine and when at least two are different are selected from the group consisting of fluorine, chlorine and bromine, with a dehydrating agent, produces a lactone of the general formula

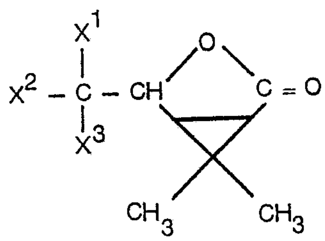

which can be isolated and converted using a reducing agent, such as zinc and acetic acid, manganese, or a zinc-copper couple, into the corresponding *cis*-diahovinylcyclopropane carboxylic acid. The two step reaction can of couse only be used to prepare a *cis*-compound from a *cis*-starting material, since the intermediate lactone cannot exist in *trans* configuration.

The Applicants have now found that this conversion can be carried out using a single step process. Moreover, this single-step process is applicable to the preparation of both *cis* and *trans* compounds.

The invention provides a process for the preparation of a compound of the general formula

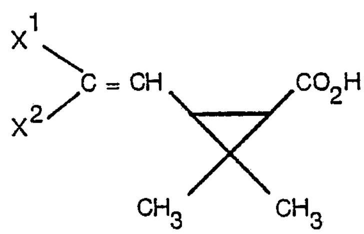

(I)

in which each of $X^1$ and $X^2$ independently represents a fluorine, chlorine or bromine atom, which comprises reacting a compound of the general formula

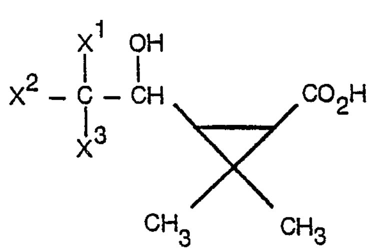

(II)

in which $X^1$ and $X^2$ have the meanings given above and $X^3$ represents a chlorine, bromine or iodine atom, with a compound of the general formula $PHal_3$ in which each Hal independently represents a chlorine or bromine atom in the presence of zinc.

The substituents $X^1$ and $X^2$ may be the same or different, but are preferably the same. Preferably $X^1$ and $X^2$ both represent bromine atoms or, especially, chlorine atoms. If two or three of $X^1$, $X^2$ and $X^3$

represent different halogen atoms, it is the halogen atom of highest molecular weight which is removed during the process. Thus, for example, to prepare a compound of the general formula I in which both $X^1$ and $X^2$ represent bromine atoms, a starting material is used in which $X^3$ also represents a bromine atom or represents an iodine atom, while to prepare a compound of the general formula I in which both $X^1$ and $X^2$ represent chlorine atoms, a starting material in which $X^3$ represents either a chlorine or a bromine or an iodine atom may be used. Preferably $X^1$, $X^2$ and $X^3$ all represent chlorine atoms.

In the compound $PHal_3$, the Hal s may be the same or different, but are preferably the same. Preferably they are all chlorine atoms.

The compounds of the general formula I and II exist in the form of optical and geometric isomers, and generally, the configuration present in the starting material is retained in the product I after the process of the invention has been carried out. Thus a starting material in which the $X^1X^2X^3C.CH.OH$- and -COOH groups are *cis* or *trans* to each other may be converted into the compound of formula I with corresponding geometry. Mixtures of isomers can of course be used. Similarly, the use of an optically active starting material can lead to an optically active product, for example one having the *R*-configuration about the cyclopropane carbon atom bearing the -COOH group.

It is essential that the reaction of the compound of the general formula II with the phosphorpous trihalide occurs in the presence of zinc. Thus for example the three reactants may be admixed simultaneously, or the zinc may be admixed with the compound of general formula II before the addition of the phosphorus trihalide. If the phosphorus trihalide is permitted to react with the compound of the general formula II without zinc being present then complex reactions may occur. For example, lactone formation may occur as described in US Patent Specification No. 4166063 if the $X^1X^2X^3C.CH.OH$-and -COOH groups in the starting material are *cis* to each other, while anhydride formation may occur if these groups are *trans* to each other. Once such a reaction has occurred, the resulting product cannot be converted satisfactorily into the desired diahalovinyl compound by reaction with zinc alone.

The process according to the invention is suitably carried out in the presence of a inert aprotic solvent, for example an amide such as dimethylformamide or dimethylacetamide; a hydrocarbon such as toluene or pentane; a ketone such as acetone; an ether such as tetrahydrofuran; sulpholane, N,N-dimethylpyrrolidone; or dimethylsulphoxide. Mixtures of solvents are often suitable.

The reaction is suitably carried out at a temperature in the range −10 to 100°C, especially 0 to 50°C. Room temperature is often most convenient.

The molar ratios of the reactants used are not critical. In general, it is preferred to use at least one, preferably at least two, moles of zinc, preferably in powder form, and at least one mole of phosphorus trihalide, per mole of the compound of the general formula II. Preferably the molar ratio of zinc to phosphorous trihalide is in the range 1:1 to 5:1, especially 1.5:1 to 2.5:1, and preferably the molar ratio of phosphorus trihalide to the compound of formula II is in the range 1:1 to 5:1, especially 1:1 to 2:1.

The starting material of the general formula II may be prepared by any suitable method. Conveniently, a keto acid of the general formula

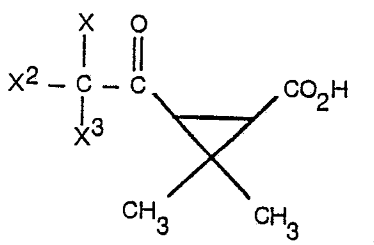

(III)

in which $X^1$, $X^2$ and $X^3$ have the meanings given above, is reduced with a reducing agent capable of selectively reducing the keto group. This reduction may for example be carried out using sodium borohydride in an aqueous medium at room temperature or below. The *cis* or *trans* relationship of the groups on the cyclopropane ring is maintained on reduction. If a *cis* keto acid is used, this generally exists in equilibrum with its lactol tautomer which has the formula

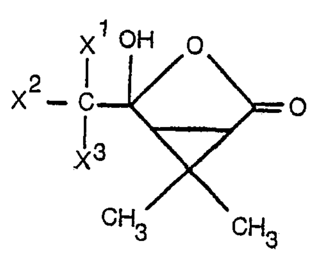

This tautomerism does not however affect the reduction.

Under suitable conditions, it is possible to prepare the compound of the general formula II as

described above, and then, without intermediate isolation, to convert it *in situ* by the process according to the invention into the compound of the general formula I.

The following Examples illustrate the invention. NMR data are given in units of ppm using tetramethyl silane as standard. The preparation of starting materials and a precursor thereof are also exemplified below, these preparations being labelled *A*, *B* and *C*.

*A Preparation of cis 2-trichloroacetyl-3,3-dimethylcyclopropane carboxylic acid and its lactol tautomer*

A suspension of sodium trichloroacetate (0.74 g, 4.0 mmol) in 10 ml acetonitrile and caronic anhydride (0.5 g) was stirred for 20 hours at room temperature. The reaction mixture was then acidified with 0.5 ml concentrated HCl, diluted with water and extracted with dichloromethane. The extracts were washed with water, dried over magnesium sulphate, filtered and evaporated to dryness. 0.8 g of a white solid were obtained. A solution of this solid in $CDCl_3$ was shown by NMR to contain a mixture of *cis* 2-trichloroacetyl-3,3-dimethylcyclopropane carboxylic acid and its lactol tautomer, 4-hydroxy-4-trichloromethyl-6,6-dimethyl-3-oxabicyclo-[3.1.0]hexan-2-one.

*B Preparation of cis 2-(2,2,2-trichloro-1-hydroxyethyl)-3,3-dimethylcyclopropane carboxylic acid*

A mixture of 35 mg sodium borohydride and 120 mg sodium bicarbonate was added while stirring over 5 minutes to 0.5 g of the product obtained in Preparation *A* in 5 ml water. 1 ml dichloromethane was added, and the mixture was then stirred for $\frac{1}{2}$ hour at room temperature. The mixture was then acidified with concentrated hydrochloroic acid and the resulting percipitate, after filtering and drying, weighed 0.29 g. NMR using hexaduetero acetone as solvent showed the product to be the desired hydroxy acid with purity greater then 95%, and also showed that the reduction had proceeded stereoselectively. Only two of the four possible isomers were present-viz. the 1*R*1'*S* and 1*S*1'*R* isomers, the 1 and 1' carbon atoms being marked in the following diagram:

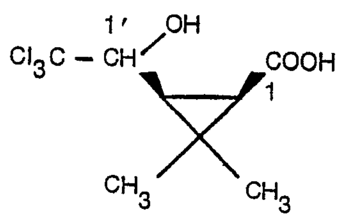

Proton NMR: 1.28 (singlet, 3H)
1.42 (singlet, 3H)
1.63 (doublet, 1H) coupling constant J = 9Hz
1.85 (double doublet, 1H) J = 9Hz
4.92 (doublet, 1H) J = 9Hz
5.5 (broad singlet, 2H)

*C Preparation of cis 2-(1-hydroxy-2,2,2-tribromoethyl)-3,3-dimethylcyclopropane carboxylic acid*

*Cis* 2-tribromoacetyl-3,3-dimethylcyclopropane carboxylic acid (0.2 mmol) prepared by a method analogous to that described in Preparation *A* was stirred for 10 minutes with a solution of sodium borohydride (0.8 mmol) in 5 ml water at room temperature. After acidification with concentrated hydrochloric acid and dilution with water, the crystalline reaction product was filtered off and dried under reduced pressure at 40°C. The resulting product weighed 13 mg and was shown by NMR to contain approximately 50% of the desired product, as a mixture of 1*R*1'*S* and 1*S*1'*R* isomers, the remaining 50% being mainly the *endo* isomer of 4-dibromomethyl-6,6-dimethyl-3-oxabicyclo[3.1.0]hexan-2-one.

Example 1

*Preparation of cis 2-(2,2-dichlorovinyl)-3,3-dimethylcyclopropane carboxylic acid*

An NMR tube was charged with 0.16 mmol of 2-(2,2,2-trichloro-1-hydroxyethyl)-3,3-dimethylcyclopropane carboxylic acid and 0.32 mmol of zinc powder in 0.4 mmol dimethylformamide. After cooling to 0°C, 0.18 mmol phosphorous trichloride were added and the mixture was stirred for 15 minutes. It was then diluted with water, acidified with concentrated hydrochloric acid and extracted with deuterochloroform. The extract was washed with water. NMR analysis showed that the desired product which has been obtained had a purity of 90%, the NMR data being as follows:—

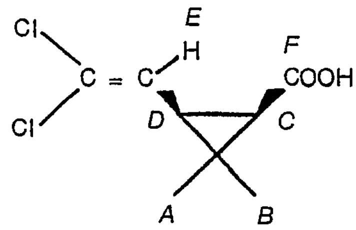

*A* and *B* 1.28 (singlet, 6H)
*C* 1.83 (doublet, 1H) $J_{CD}$= 8Hz
*D* 2.04 (double doublet, 1H) $J_{DE}$= 8.5Hz
*E* 6.27 (doublet, 1H)
*F* 10.90 (singlet, 1H)

Example 2

*Preparation of cis 2-(2,2-dibromovinyl)-3,3-dimethylcyclopropane carboxylic acid*

An NMR tube was charged with 0.167 mmol of 2-(2,2,2-tribromo-1-hydroxyethyl)-3,3-dimethylcyclopropane carboxylic acid and 0.33 mmol of zinc powder in 0.4 ml dimethylformamide. Afterwards, 0.183 mmol phosphorus trichloride was added at 0°C and the reaction mixture was shaken for one minute. It was then diluted with water and extracted with deuterochloroform. The extract was washed with water and analysed by NMR, which showed that the desired product has been obtained with 70% purity, the NMR data being as follows:—

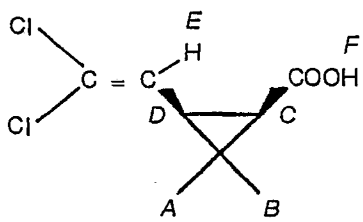

*A* and *B* 1.33 (singlet, 6H)
*C* 1.87 (doublet, 1H) $J_{CD}$ = 9Hz
*D* 2.03 (double doublet, 1H) $J_{DE}$ = 8.5Hz
*E* 6.77 (doublet, 1H)
*F* 10.83 (singlet, 1h)

**Claims**

1. A process for the preparation of a compound of the general formula

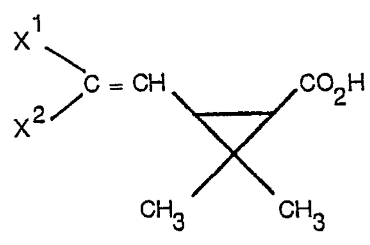

(I)

in which each of $X^1$ and $X^2$ independently represents a fluorine, chlorine or bromine atom characterised in that a compound of the general formula

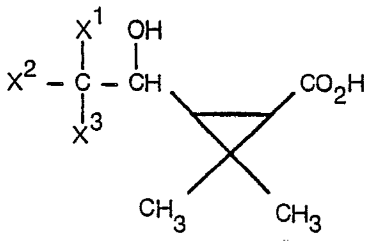

(II)

in which $X^1$ and $X^2$ have the meanings given above and $X^3$ represents a chlorine, bromine or iodine

atom is reacted with a compound of the general formula $PHal_3$ in which each Hal independently represents a chlorine or bromine atome, in the presence of zinc.

2. A process according to Claim 1, characterised in that $X^1$ and $X^2$ both represent bromine or chlorine atoms.

3. A process according to Claim 1 or 2 characterised in that all Hal's represent chlorine atoms.

4. A process according to any one of claims 1 to 3 characterised in that the reaction temperature is in the range 0 to 50°C.

5. A process according to any one of claims 1 to 4, characterised in that at least two moles of zinc and at least one mole of the compound $PHal_3$ are used per mole of compound of the general formula II.

**Revendications**

1. Un procédé pour la préparation d'un composé de la formule générale

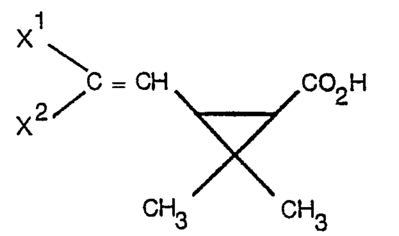

(I)

dans laquelle $X^1$ et $X^2$ représentent chacun indépendamment un atome de fluor, de chlore ou de brome, caractérisé en ce qu'un composé de la formule générale

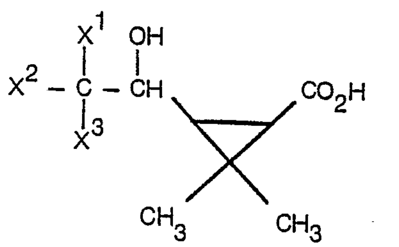

(II)

dans laquelle $X^1$ et $X^2$ ont les significations indiquées ci-dessus et $X^3$ représente un atome de chlore, de brome ou d'iode, est mis à réagir avec un composé de la formule générale $PHal_3$ dans laquelle chaque Hal indépendamment représente un atome de chlore ou de brome, en présence de zinc.

2. Un procédé selon la revendication 1, caractérisé en ce que $X^1$ et $X^2$ représentent tous deux des atomes de brome ou de chlore.

3. Un procédé selon la revendication 1 ou 2, caractérisé en ce que tous les Hal représentent des atomes de chlore.

4. Un procédé selon l'une quelconque des revendications 1 à 3, caractérisé en ce que la température de réaction est comprise entre 0 et 50°C.

5. Un procédé selon l'une quenconque des revendications 1 à 4, caractérisé en ce qu'on utilise au moins deux moles de zinc et au moins une mole de composé $PHal_3$ par mole de composé de la formule générale II.

**Patentansprüche**

1. Ein Verfahren zur Herstellung einer Verbindung der allgemeinen Formel

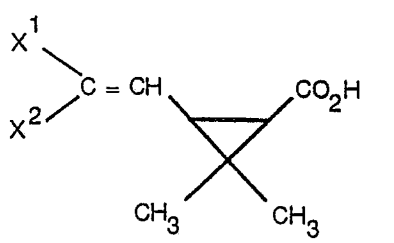

(I)

worin jeder der Substituenten $X^1$ und $X^2$ unabhängig voneinander ein Fluo-, Chlor- oder Bromatom darstellt, dadurch gekennzeichnet, daß eine Verbindung der allgemeinen Formel

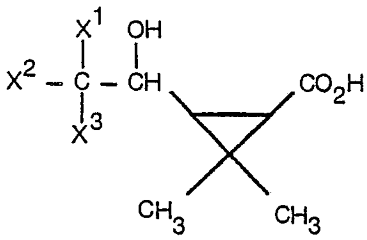

(II)

worin $X^1$ und $X^2$ die vorstehende Bedeutung aufweisen und $X^3$ für ein Chlor-, Brom oder Jodatom steht, mit einer Verbindung der allgemeinen Formel $PHal_3$, worin jedes Hal unabhängig voneinander ein Chlor- oder Bromatom bedeutet, in Gegenwart von Zink umgesetzt wird.

2. Ein Verfahren nach Anspruch 1, dadurch gekennzeichnet, daß $X^1$ und $X^2$ beide Brom- oder Chloratome bedeuten.

3. Ein Verfahren nach Anspruch 1 oder 2, dadurch gekennzeichnet, daß alle Hal-Reste Chloratome bedeuten.

4. Ein Verfahren nach einem der Ansprüche 1 bis 3, dadurch gekennzeichnet, daß die Reaktionstemperatur in dem Bereich von 0 bis 50°C liegt.

5. Ein Verfahren nach einem der Ansprüche 1 bis 4, dadurch gekennzeichnet, daß wenigstens 2 Mole Zink und wenigstens 1 Mol der Verbindung $PHal_3$ je Mol der Verbindung der allgemeinen Formel (II) verwendet werden.